# EUROPEAN PATENT APPLICATION

(11) **EP 2 907 508 A1**
(43) Date of publication of application: **19.08.2015**
(21) Application number: 15153660.4
(22) Date of filing: 03.02.2015
(51) Int. Cl.: A61K 9/20, C08B 11/193, C08B 11/20

(54) **Hydroxyalkylalkyl cellulose for tableting and solid preparation comprising the same**

(30) Priority: 14.02.2014 JP 2014026500
(71) Applicant: Shin-Etsu Chemical Co., Ltd., Chiyoda-ku Tokyo 100 (JP)
(72) Inventor: Yokosawa, Takuya, NIIGATA-KEN, Niigata 942-8601 (JP); Maruyama, Naosuke, NIIGATA-KEN, Niigata 942-8601 (JP)
(74) Representative: Vidon Brevets & Stratégie

(57) **Abstract**

Provided are a hydroxyalkylalkyl cellulose excellent in formability and not causing marked delay in disintegration when added even in a small amount; a solid preparation comprising the hydroxyalkylalkyl cellulose; and a method for producing the solid preparation. More specifically, provided are a hydroxyalkylalkyl cellulose for tableting having a specific surface area of from 0.5 to 5.0 m²/g as measured by BET and a solid preparation comprising the hydroxyalkylalkyl cellulose. Also provided is a method for producing the hydroxyalkylalkyl cellulose for tableting, comprising the steps of: bringing pulp into contact with an alkali metal hydroxide solution to obtain an alkali cellulose, reacting the alkali cellulose with an etherifying agent to obtain a first hydroxyalkylalkyl cellulose, grinding the first hydroxyalkylalkyl cellulose, and subjecting the ground first hydroxyalkylalkyl cellulose to hydrolysis in the presence of an acid catalyst or oxidative degradation in the presence of an oxidant to obtain a second hydroxyalkylalkyl cellulose.

## Description

### BACKGROUND OF THE INVENTION

### 1. Field of the invention

The present invention relates to, in the fields of pharmaceuticals and food, a hydroxyalkylalkyl cellulose exhibiting high formability when added even in a small amount and a solid preparation comprising the hydroxyalkylalkyl cellulose.

### 2. Description of the related art

In the fields of pharmaceuticals and food, a method of producing a solid preparation, particularly a tablet, includes a dry direct tableting method comprising the step of tableting a mixture of a drug and an additive as it is; and a wet granulation tableting method comprising the steps of granulating a mixture of a drug and an additive in the presence of a binder solution or a proper solvent such as water, drying and tableting. In the dry direct tableting method, when the drug or the additive has poor fluidity or formability, the mixture may be subjected to roll compression (dry granulation), crushed and then tableted (dry granulation tableting). In the wet granulation tableting method, an agitating granulator or a fluidized bed granulator may be used.

The dry direct tableting method has been employed frequently in recent years because it can be used even when a drug is sensitive to water, or its simple steps facilitate process control. Compared with the wet granulation tableting method, however, the dry direct tableting method usually requires a larger amount of the additive in order to secure formability. Examples of the additive having high formability include crystalline cellulose with high formability (JP 06-316535A), hydroxyalkyl cellulose fine particles (WO/2011/065350), and low-substituted hydroxypropyl cellulose with high formability and high fluidity (JP 2010-254756A).

On the other hand, there is a recent tendency of reducing the size of tablets to make them easier to swallow and therefore suppressing an amount of additive such as a binder. A binder capable of enhancing the hardness of tablets when added even in a small amount is thus demanded.

### SUMMARY OF THE INVENTION

The crystalline cellulose disclosed in JP 06-316535A, however, should be added in a larger amount in order to secure formability so that it is not suited for use in a solid preparation or a small-sized tablet having high drug content. The fine particles disclosed in WO/2011/065350 are inferior in disintegrability, though having excellent formability. On the other hand, the low-substituted hydroxypropyl cellulose disclosed in JP 2010-254756A is excellent in disintegrability, but does not have satisfactory formability. Thus, in the conventional art, it is difficult to secure high formability without sacrificing disintegrability when such an additive is added in a small amount.

With the foregoing in view, the present invention has been made. An object of the invention is to provide a hydroxyalkylalkyl cellulose exhibiting excellent formability when added even in a small amount and not causing marked delay in disintegration; a solid preparation comprising the hydroxyalkylalkyl cellulose; and a method for producing the solid preparation.

With a view to achieving the above-mentioned object, the present inventors have proceeded with an intensive investigation. As a result, it has been found that the object can be achieved by using a hydroxyalkylalkyl cellulose having a certain specific surface area, leading to the completion of the invention.

In one aspect of the invention, there are provided a hydroxyalkylalkyl cellulose for tableting having a specific surface area of from 0.5 to 5.0 m²/g as measured by the BET method, and a solid preparation comprising the hydroxyalkylalkyl cellulose for tableting. In another aspect of the invention, there is also provided a method for producing a hydroxyalkylalkyl cellulose for tableting having a specific surface area of from 0.5 to 5.0 m²/g as measured by the BET method, comprising the steps of: bringing pulp into contact with an alkali metal hydroxide solution to obtain an alkali cellulose, reacting the alkali cellulose with an etherifying agent to obtain a first hydroxyalkylalkyl cellulose, grinding the first hydroxyalkylalkyl cellulose, and subjecting the ground first hydroxyalkylalkyl cellulose to hydrolysis in the presence of an acid catalyst or oxidative degradation in the presence of an oxidant for depolymerization to obtain a second hydroxyalkylalkyl cellulose. In a further aspect of the invention, there is also provided a method for producing a solid preparation comprising: the steps comprised by the method to produce a hydroxyalkylalkyl cellulose for tableting and a step of tableting the hydroxyalkylalkyl cellulose by dry direct tableting or dry granulation tableting.

According to the invention, since the hydroxyalkylalkyl cellulose shows high formability, it can enhance tablet hardness when a tablet is produced by dry direct tableting or dry granulation tableting. In particular, it is effective for a formulation in which the amount of an additive is limited, for example, a formulation in which drug content should be increased, a formulation for a small-sized tablet, or a formulation for a granule-containing tablet which requires tableting at a low pressure. In addition, according to the invention, since the hydroxyalkylalkyl cellulose shows high formability, flakes are available in a high yield during dry granulation, and when the flakes are re-ground into granules or fine granules, an amount of fine powders generated is low and the obtained tablet has high tablet hardness.

### DETAILED DESCRIPTION OF THE PREFERRED EMBODIMENTS

The invention will hereinafter be described more specifically.

The hydroxyalkylalkyl cellulose has a specific surface area, as measured by the BET (BET multipoint) method, of from 0.5 to 5.0 m²/g, preferably from 0.5 to 4.0 m²/g, more preferably from 0.6 to 3.0 m²/g, still more preferably 1.0 to 3.0 m²/g. When the specific surface area is less than 0.5 m²/g, the desirable formability cannot be obtained. When the specific surface area is more than 5.0 m²/g, miscibility with a drug or fluidity are lowered when the hydroxyalkylalkyl cellulose is comprised by a tablet.

The specific surface area can be analyzed using the BET (BET multipoint) method which is based on low-temperature low-humidity physical adsorption of an inert gas, the method allowing a molecule having a known adsorption area to adsorb onto the surface of powder particles of the sample at a temperature of liquid nitrogen and determining a specific surface area of the sample from the adsorption amount. It can be measured in accordance with "Method 2: The volumetric method" in "Specific Surface Area by Gas Adsorption" in General Tests of the Japanese Pharmacopoeia Sixteenth Edition, and for example, using an automatic surface area and porosimetry analyzer "TriStar II 3020" (trade name; product of Micromeritics Instrument Corporation).

The viscosity at 20°C of 2% by weight aqueous solution of the hydroxyalkylalkyl cellulose is preferably from 1 to 15 mPa·s, more preferably from 2 to 6 mPa·s, still more preferably from 2.5 to 4.5 mPa·s. When the viscosity is less than 1 mPa·s, disintegrability may be deteriorated. When the viscosity is more than 15 mPa·s, not only disintegrability but also formability may be deteriorated, thereby resulting in failure of increasing tablet hardness. As described in JP 06-316535A or WO 2011/065350, it is known that a polymer having a lower polymerization degree (i.e. a lower viscosity) usually has lower formability. However, it is surprising that the present inventors have found that a hydroxyalkylalkyl cellulose having a lower polymerization degree is superior in formability. It is presumed that a hydroxyalkylalkyl cellulose having a lower viscosity (i.e. a lower polymerization degree) is likely to undergo rearrangement of molecular chains and cause plastic deformation easily during compression so that formability is improved and tablet hardness is increased.

With respect to the above viscosity, the viscosity of 600 mPa·s or greater can be measured using a B type viscometer in accordance with JIS Z8803, while the viscosity of less than 600 mPa·s can be measured using an Ubbelohde viscometer in accordance with JIS K2283-1993.

The hydroxyalkylalkyl cellulose has an average particle size of preferably from 5 to 70 µm, more preferably from 10 to 50 µm, still more preferably from 10 to 30 µm. When the average particle size is more than 70 µm, an adequate specific surface area may not be secured so that desired formability may not be obtained. When the average particle size is less than 5 µm, miscibility with a drug or fluidity may be lowered when the hydroxyalkylalkyl cellulose is comprised by a tablet.

The average particle size means a volume-based average particle size and as described, for example, in page 88 of "Kaitei Zoho Funtai Bussei Zusetsu (revised and enlarged edition, Physical Properties of Powder with Illustrations" edited by The Society of Powder Technology, Japan, and The Association of Powder Method Industry and Engineering, Japan, published by Nikkei Gijutsu Tosho Co., Ltd., 1985, it is calculated using the formula: {∑(nD³)/∑n}^{1/3} wherein D is a particle diameter, n is the number of particles each having the particle diameter D, and ∑n is a total number of particles. The term "D₅₀" means a particle size (average particle size) when the cumulative particle-size distribution is 50%. The average particle size can be measured using a dry laser diffraction method. For example, a volume-average particle size can be determined from a diffraction intensity obtained by irradiating a laser light to a powder sample ejected by compressed air, as in the method using "Mastersizer 3000" (trade name; product of Malvern in England) or "HELOS" (trade name; product of Sympatec in Germany).

The hydroxyalkylalkyl cellulose has a loose bulk density of preferably from 0.05 to 0.5 g/mL, more preferably from 0.1 to 0.4 g/mL, still more preferably from 0.1 to 0.3 g/mL. When the loose bulk density is less than 0.05 g/mL, miscibility with a drug or fluidity may be lowered when the hydroxyalkylalkyl cellulose is comprised by a tablet. When the loose bulk density is more than 0.5 g/mL, formability may be deteriorated.

The term "loose bulk density" means a bulk density in a loosely filled state and is determined by evenly charging a cylindrical vessel made of a stainless steel and having a diameter of 5.03 cm and a height of 5.03 cm (capacity: 100 ml) with a sample through a JIS 22-mesh sieve having opening of 710 µm from 23 cm above the vessel, leveling off the top surface of the sample, and then weighing.

The hydroxyalkylalkyl cellulose is a nonionic polymer obtained by etherifying some of the hydroxyl groups on the glucose ring of cellulose. Examples of the hydroxyalkylalkyl cellulose include hydroxypropylmethyl cellulose and hydroxyethylmethyl cellulose. Of these, hydroxypropylmethyl cellulose is particularly preferred from the standpoint of formability and disintegrability.

The degree of substitution of the hydroxyalkylalkyl cellulose is not particularly limited. For example, hydroxypropylmethyl cellulose has the degree of methoxy substitution of preferably from 16.5 to 30.0% by weight, more preferably from 19.0 to 30.0% by weight; and has the degree of hydroxypropoxy substitution of preferably from 3.0 to 32.0% by weight, more preferably from 3.0 to 12.0% by weight. These degrees of substitution can be measured using a method based on the assay of the degree of substitution of hydroxypropylmethyl cellulose (hypromellose) in the Japanese Pharmacopoeia Sixteenth Edition.

Next, a solid preparation comprising the hydroxyalkylalkyl cellulose will be described.

Since the hydroxyalkylalkyl cellulose has high formability, addition of the hydroxyalkylalkyl cellulose even in a small amount can enhance tablet hardness when a tablet is produced by the dry direct tableting or the dry granulation tableting. Although the meaning of the term "small" in the "small amount" differs depending on the weight or shape of the tablet or a type of drug comprised by the tablet, hydroxyalkylalkyl cellulose content in the solid preparation is preferably 20% by weight or less, more preferably 10% by weight or less, still more preferably 5% by weight or less. When the content is more than 20% by weight, a disintegration time may be deteriorated, though tablet hardness increases. Although the lower limit of the hydroxyalkylalkyl cellulose content differs depending on the weight or shape of the tablet or a type of drug comprised by the tablet, the hydroxyalkylalkyl cellulose content is preferably 0.1% by weight or more, more preferably 1% by weight or more. When the content is less than 0.1% by weight, desired formability may not be obtained.

Next, a method for producing the hydroxyalkylalkyl cellulose will be described.

An alkali metal hydroxide solution and pulp are brought into contact with each other by a conventional method to obtain alkali cellulose. The pulp may be in sheet form or chip form, preferably in powder form obtained by grinding in a grinder. The step of bringing the pulp into contact with an alkali metal hydroxide solution may be preferably carried out in a reactor having an internal stirring structure.

The alkali cellulose thus obtained is reacted with an etherifying agent by a conventional method to obtain a first (high-polymerization-degree, pre-depolymerization) hydroxyalkylalkyl cellulose.

The etherifying agent useful for producing the first hydroxyalkylalkyl cellulose is known and not particularly limited. Examples of the etherifying agent include an alkylating agent such as methyl chloride, and a hydroxyalkylating agent such as propylene oxide or ethylene oxide.

The first hydroxyalkylalkyl cellulose obtained by the etherification reaction may be optionally purified and/or dried by a conventional method.

A purification method or an apparatus to be used for purification is not particularly limited. The purification method or an apparatus is preferably a washing method or washing apparatus using preferably water, more preferably hot water of preferably from 85 to 100°C.

A drying method or an apparatus to be used for drying is not particularly limited. The drying method or an apparatus is preferably a method or apparatus capable of setting the temperature of the first hydroxyalkylalkyl cellulose during drying at from 40 to 80°C.

The viscosity at 20°C of 2% by weight aqueous solution of the first hydroxyalkylalkyl cellulose, optionally after the purification and/or drying, is preferably more than 20 mPa·s, more preferably from 50 to 200,000 mPa·s, still more preferably from 100 to 10,000 mPa·s, particularly preferably from 100 to 3000 mPa·s. Regarding the viscosity at 20°C of 2% by weight aqueous solution of the first hydroxyalkylalkyl cellulose, the viscosity of 600 mPa·s or greater can be measured using a B type viscometer in accordance with JIS Z8803, while the viscosity of less than 600 mPa·s can be measured using an Ubbelohde viscometer in accordance with JIS K2283-1993.

The first hydroxyalkylalkyl cellulose, optionally after the purification and/or drying, is ground into particles having an average particle size of preferably from 5 to 200 µm, more preferably from 10 to 100 µm in order to have the above-mentioned specific surface area of hydroxyalkylalkyl cellulose as a final product. After the grinding, the resulting particles may be optionally classified through a sieve having a predetermined opening size to control the specific surface area.

A grinding method or an apparatus to be used for grinding is not particularly limited. The apparatus is preferably an impact grinder such as "Turbo Mill" (trade name; product of Freund-Turbo Corporation), "PPSR" (trade name; product of Pallmann Industries), "Victory Mill" (trade name; product of Hosokawa Micron) and "Jet Mill" (trade name; product of Nippon Pneumatic Mfg Co., Ltd.); or a compaction mill such as a vibration mill, a ball mill, a roller mill, and a bead mill, so as to obtain a high specific surface area.

Next, the first hydroxyalkylalkyl cellulose is depolymerized into a second (low-polymerization-degree, post-depolymerization) hydroxyalkylalkyl cellulose. Further improvement in formability can be expected from the depolymerization that decreases a polymerization degree. Depolymerization is carried out by hydrolysis in the presence of an acid catalyst or oxidative degradation in the presence of an oxidant. The depolymerization is carried out preferably by hydrolysis in the presence of an acid catalyst.

Examples of an acid to be used for the depolymerization by hydrolysis in the presence of an acid catalyst preferably include an inorganic acid such as hydrochloric acid, sulfuric acid, nitric acid and phosphoric acid. The acid may be used singly or in combination of two or more acids.

The acid to be added to the system is in a gas form or a solution form. The acid is added preferably in a solution form. The amount of the acid to be added is preferably from 0.1 to 3.0% by weight, more preferably from 0.15 to 1.5% by weight relatively on basis of the weight of the hydroxyalkylalkyl cellulose.

An internal temperature during the depolymerization is not particularly limited, and is preferably from 50 to 130°C, more preferably from 60 to 110°C, still more preferably from 60 to 90°C. The depolymerization time is preferably set based on the respective viscosities at 20°C of 2% by weight aqueous solutions of the first (pre-depolymerization) hydroxyalkylalkyl cellulose and the second (post-depolymerization) hydroxyalkylalkyl cellulose and depolymerization conditions.

The viscosity of 2% by weight aqueous solution of the second hydroxyalkylalkyl cellulose obtained after the depolymerization is preferably from 1 to 15 mPa·s.

When the second hydroxyalkylalkyl cellulose thus obtained has a specific surface area outside the range of the invention, the second hydroxyalkylalkyl cellulose can be further ground to obtain a hydroxyalkylalkyl cellulose having the specific surface area of the invention. The specific surface area may be controlled by classifying the ground hydroxyalkylalkyl cellulose through a sieve or the like having a predetermined opening size. A grinding method or an apparatus to be used for grinding is not particularly limited and the above-mentioned apparatus or the like can be used. The sieve to be used for classification is not particularly limited, and preferably includes a sieve such as a JIS 200-mesh sieve having opening of 75 µm, a JIS 235-mesh sieve having opening of 63 µm, a JIS 330-mesh sieve having opening of 45 µm, a JIS 390-mesh sieve having opening of 38 µm.

Next, a method for producing a solid preparation comprising the resulting hydroxyalkylalkyl cellulose for tableting will be described.

A solid preparation can be obtained by tableting or granulating the hydroxyalkylalkyl cellulose for tableting, together with a drug and a various type of additive used commonly in this field such as an excipient, a disintegrant, a binder, an aggregation-preventing agent, or a solubilizing agent for a pharmaceutical compound. Examples of the solid preparation include a tablet, a granule, a powder, and a capsule.

Of the above, a tablet can be produced by any of dry direct tableting, dry granulation tableting, wet agitation granulation tableting, and fluidized bed granulation tableting. The dry direct tableting and the dry granulation tableting are particularly preferred because they do not need dissolution of the hydroxyalkylalkyl cellulose.

The dry direct tableting is a method comprising the steps of dry-mixing the hydroxyalkylalkyl cellulose for tableting, a drug, an optional excipient, an optional disintegrant, an optional lubricant and the like to obtain a mixture and tableting the mixture. Since the dry direct tableting does not comprise a granulation step, the production method can be simplified. Thus, the dry direct tableting is a highly productive method.

The dry granulation tableting is a method comprising the steps of compression-granulating the hydroxyalkylalkyl cellulose for tableting, a drug, an optional excipient, an optional disintegrant, an optional lubricant and the like to obtain granules and tableting the granules. The dry granulation tableting is a method effective for a drug sensitive to water or solvent. The granules can be obtained, for example, through roller compression by using a compaction granulator such as roller compactor. The roller pressure differs depending on the physical properties of the powder, and is preferably from 1 to 30 MPa, more preferably from 2 to 12 MPa. The rotation speed of the roller is preferably from 1 to 50 rpm, more preferably from 2 to 20 rpm. The rotation speed of a screw is preferably from 1 to 100 rpm, more preferably from 2 to 50 rpm. Flakes obtained by roller compression are ground and sized into tableting powders by using a grinder or crusher such as a comil, a quick mill or a power mill.

The hydroxyalkylalkyl cellulose for tableting can also be used for orally disintegrating tablets that have been investigated actively in recent years.

A drug to be comprised by a solid preparation comprising the hydroxylalkylalkyl cellulose of the invention is not particularly limited insofar as it is orally administrable. Examples of such a drug include drugs for central nervous system; drugs for cardiovascular system; drugs for respiratory system; drugs for digestive system; antibiotics; antitussives and expectorants; antihistamines; analgesic, antipyretic and anti-inflammatory drugs; diuretics; autonomic drugs; antimalarial drugs; antidiarrheal agents; psychotropic drugs; and vitamins and derivatives thereof.

Examples of the drugs for central nervous system include diazepam, idebenone, aspirin, ibuprofen, paracetamol, naproxen, piroxicam, dichlofenac, indomethacin, sulindac, lorazepam, nitrazepam, phenytoin, acetaminophen, ethenzamide, ketoprofen, and chlordiazepoxide.

Examples of the drugs for cardiovascular system include molsidomine, vinpocetine, propranolol, methyldopa, dipyridamol, furosemide, triamterene, nifedipine, atenolol, spironolactone, metoprolol, pindolol, captopril, isosorbide nitrate, delapril hydrochloride, meclofenoxate hydrochloride, diltiazem hydrochloride, etilefrine hydrochloride, digitoxin, propranolol hydrochloride, and alprenolol hydrochloride. Examples of the drugs for respiratory system include amlexanox, dextromethorphan, theophilline, pseudo-ephedrine, salbutamol, and guaiphenecin.

Examples of the drugs for digestive system include benzimidazole-based drugs having anti-ulcer action such as 2-[(3-methyl-4-(2,2,2-trifluoroethoxy)-2-pyridyl)methylsulfinyl]benzimidazole and 5-methoxy-2-[(4-methoxy-3,5-dimethyl-2-pyridyl)methylsulfinyl]benzimidazole, cimetidine, ranitidine, pirenzepine hydrochloride, pancreatin, bisacodyl, and 5-aminosalicylic acid.

Examples of the antibiotics include talampicillin hydrochloride, bacampicillin hydrochloride, cephaclor, and erythromycin.

Examples of the antitussives and expectorants include noscapine hydrochloride, carbetapentane citrate, dextromethorphan hydrobromide, isoaminile citrate, and dimemorfan phosphate.

Examples of the antihistamines include chlorpheniramine maleate, diphenhydramine hydrochloride, and promethazine hydrochloride.

Examples of the analgesic, antipyretic and anti-inflammatory drugs include ibuprofen, diclofenac sodium, flufenamic acid, sulpyrine, aspirin, and ketoprofen.

Examples of the diuretics include caffeine.

Examples of the autonomic drugs include dihydrocodeine phosphate, dl-methylephedrine hydrochloride, atropine sulfate, acetylcholine chloride, and neostigmine.

Examples of the antimalarial drugs include quinine hydrochloride.

Examples of the antidiarrheal agents include loperamide hydrochloride. Examples of the psychotropic drugs include chlorpromazine.

Examples of the vitamins and derivatives thereof include Vitamin A, Vitamin B1, fursultiamine, Vitamin B2, Vitamin B6, Vitamin B12, Vitamin C, Vitamin D, Vitamin E, Vitamin K, calcium pantothenate, and tranexamic acid.

Examples of the excipient include sugars such as sucrose, lactose, and glucose; sugar alcohols such as mannitol, sorbitol, and erythritol; starches; crystalline cellulose; calcium phosphate; and calcium sulfate.

Examples of the binder include polyvinyl alcohol, polyacrylic acid, polymethacrylic acid, polyvinylpyrrolidone, glucose, sucrose, lactose, maltose, dextrin, sorbitol, mannitol, macrogols, gum arabic, gelatin, agar, and starches, crystalline cellulose, and low-substituted hydroxypropyl cellulose,

Examples of the disintegrant include low-substituted hydroxypropyl cellulose, carmellose and salt thereof, croscarmellose sodium, carboxymethyl starch sodium, crospovidone, crystalline cellulose, and crystalline cellulose carmellose sodium.

Examples of the lubricant and the aggregation-preventing agent include talc, magnesium stearate, calcium stearate, colloidal silica, stearic acid, waxes, hydrogenated oils, polyethylene glycols, and sodium benzoate.

Examples of the solubilizing agent for a pharmaceutical compound include organic acids such as fumaric acid, succinic acid, malic acid, and adipic acid.

### EXAMPLES

The invention will hereinafter be described more specifically by Examples. It should not be construed that the invention is limited to or by these Examples. Modification in various ways can be made by those skilled in the art within the technical concept of the invention.

### <Example 1>

Powdery pulp having 6.0 kg in terms of cellulose content was placed in an internal stirring type pressure reactor. After vacuuming, 15.4 kg of 49% by weight aqueous sodium hydroxide solution was added thereto and stirred to obtain alkali cellulose. Then, 12.4 kg of methyl chloride and 2.8 kg of propylene oxide were added thereto, reacted with the alkali cellulose, washed, dried, and ground to obtain first (high-polymerization-degree, pre-depolymerization) hydroxypropylmethyl cellulose. The resulting first hydroxypropylmethyl cellulose had a degree of methoxy substitution of 29% by weight, a degree of hydroxypropoxy substitution of 10% by weight and a specific surface area of 0.24 m²/g, and a 2% by weight aqueous solution thereof had a viscosity at 20°C of 1510 mPa·s.

The first hydroxypropylmethyl cellulose was subjected to addition of a 12% by weight aqueous hydrochloric acid solution in such an amount as to have a concentration of 0.3% by weight on basis of the first hydroxypropylmethyl cellulose. The internal temperature was controlled to 81°C and depolymerization was carried out for 200 minutes to obtain a second (low-polymerization-degree, post-depolymerization) hydroxypropylmethyl cellulose. The second hydroxypropylmethyl cellulose thus obtained was ground using a high-speed rotating impact grinder "Victory Mill" equipped with a screen of 0.3-mm opening to obtain intended ground second hydroxypropylmethyl cellulose. The resulting powder had a specific surface area, average particle size, loose bulk density, and viscosity at 20°C in 2% by weight aqueous solution as shown in Table 1.

Next, components of the following tablet composition except magnesium stearate were mixed for 3 minutes in a plastic bag, and then subjected to addition of magnesium stearate and subsequent mixing for 30 seconds. The resulting mixture was subjected to the dry direct tableting under the following tableting conditions to obtain tablets. The tablets thus obtained were evaluated for tablet hardness and disintegration time. The results are shown in Table 1.

### Composition of Tablet

Acetaminophen fine powder (product of Yamamoto Corporation): 50.0 parts by weight
Lactose hydrate (trade name "Dilactose S", product of Freund Corp.): 44.5 parts by weight
Hydroxypropylmethyl cellulose: 5.0 parts by weight
Light silicic anhydride: 0.5 part by weight
Magnesium stearate: 0.5 part by weight

### Tableting conditions

Tableting machine: rotary tableting machine (trade name "VIRGO"; product of Kikusui Seisakusho)
Tablet size: 200 mg/tablet, a diameter (D) of 8 mm and radius of curvature (R) of 12 mm
Tableting pressure: 10 kN
Tableting speed: 20 rpm

### <Measurement conditions>

The specific surface area by the BET (BET multipoint) method was measured in accordance with "Method 2: The volumetric method" in "Specific Surface Area by Gas Adsorption" in General Tests of the Japanese Pharmacopoeia Sixteenth Edition. It was measured through a gas adsorption method in which an adsorption gas of nitrogen and a refrigerant of liquid nitrogen were employed within the range of relative pressure (P/Pₒ) of 0.05 to 0.30 wherein Pₒ represents a saturated vapor pressure and P represents a measurement equilibrium pressure, by using an automatic specific surface area and porosimetry analyzer "TriStar II 3020" (trade name; product of Micrometrics Instrument Corporation). The sample was absolutely dried before the measurement since the sample had been left at 105°C for two hours. An amount of sample for the measurement was varied within the range of 0.5 to 2 g depending on the type of sample.

The viscosity at 20°C of 2% by weight aqueous solution was measured as follows. When the viscosity was 600 mPa·s or greater, it was measured using a B type viscometer in accordance with JIS Z8803. When the viscosity was less than 600 mPa·s, it was measured using an Ubbelohde viscometer in accordance with JIS K2283-1993.

The average particle size was measured through laser diffraction having Fraunhofer approximation at dispersion pressure of 2 to 3 bar and at scattered intensity of 2 to 10% using "Mastersizer 3000" (trade name; product of Malvern in England).

The loose bulk density was measured using a powder tester "PT-S" (product of Hosokawa Micron). A sample was supplied uniformly into a cylindrical vessel made of stainless steel and having a diameter of 5.03 cm and a height of 5.03 cm (capacity: 100 ml) from 23 cm above the vessel through a JIS 22-mesh sieve having opening of 710 µm, and was weighed after leveling off the top surface of the sample.

The tablet hardness was measured using a tablet hardness tester (trade name "TBH-125"; product of ERWEKA GmbH). A load was applied to the diameter direction of a tablet at a rate of 1 mm/sec and the maximum breaking strength at which the tablet was broken was measured.

The disintegration time was measured by using pure water as a test liquid in accordance with the Japanese Pharmacopoeia, Sixteenth Edition.

### <Example 2>

The ground second hydroxypropylmethyl cellulose obtained in Example 1 was sieved through a sieve having a 38 µm-opening to obtain intended hydroxypropylmethyl cellulose powder. The physical properties of the powder and physical properties of tablets obtained by the dry direct tableting in the same manner as in Example 1 are shown in Table 1.

### <Example 3>

The first (high-polymerization-degree, pre-depolymerization) hydroxypropylmethyl cellulose was obtained in the same manner as in Example 1 except that 8.2 kg of 49% by weight aqueous sodium hydroxide solution, 7.1 kg of methyl chloride and 1.6 kg of propylene oxide were used. The resulting first hydroxypropylmethyl cellulose had a degree of methoxy substitution of 22% by weight, a degree of hydroxypropoxy substitution of 9% by weight.

The first hydroxypropylmethyl cellulose was subjected to addition of a 12% by weight aqueous hydrochloric acid solution in such an amount as to have a concentration of 0.3% by weight on basis of the first hydroxypropylmethyl cellulose. The internal temperature was controlled to 79°C and depolymerization was carried out for 160 minutes to obtain a second (low-polymerization-degree, post-depolymerization) hydroxypropylmethyl cellulose. The second hydroxypropylmethyl cellulose thus obtained was ground using an air stream impact grinder "Jet Mill" (product of Nippon Pneumatic Mfg Co., Ltd.) to obtain intended ground second hydroxypropylmethyl cellulose. The powder properties of the ground second hydroxypropylmethyl cellulose and tablet properties of the tablet obtained by the dry direct tableting in the same manner in Example 1 are shown in Table 1.

### <Example 4>

The first (high-polymerization-degree, pre-depolymerization) hydroxypropylmethyl cellulose was obtained in the same manner as in Example 1 except that 15.2 kg of 49% by weight aqueous sodium hydroxide solution, 12.2 kg of methyl chloride and 1.7 kg of propylene oxide were used. The resulting first hydroxypropylmethyl cellulose had a degree of methoxy substitution of 29.5% by weight, a degree of hydroxypropoxy substitution of 6% by weight.

The first hydroxypropylmethyl cellulose was subjected to addition of a 12% by weight aqueous hydrochloric acid solution in such an amount as to have a concentration of 0.3% by weight on basis of the first hydroxypropylmethyl cellulose. The internal temperature was controlled to 79°C and depolymerization was carried out for 140 minutes to obtain a second (low-polymerization-degree, post-depolymerization) hydroxypropylmethyl cellulose. The second hydroxypropylmethyl cellulose thus obtained was ground using an air stream impact grinder "Jet Mill" (product of Nippon Pneumatic Mfg Co., Ltd.) to obtain intended ground second hydroxypropylmethyl cellulose. The powder properties of the ground second hydroxypropylmethyl cellulose and tablet properties of the tablet obtained by the dry direct tableting in the same manner in Example 1 are shown in Table 1.

### <Example 5>

The first (high-polymerization-degree, pre-depolymerization) hydroxypropylmethyl cellulose was obtained in the same manner as in Example 1 except that 12.4 kg of 49% by weight aqueous sodium hydroxide solution, 10.0 kg of methyl chloride and 0.8 kg of propylene oxide were used. The resulting first hydroxypropylmethyl cellulose had a degree of methoxy substitution of 29.5% by weight, a degree of hydroxypropoxy substitution of 3.1% by weight.

The first hydroxypropylmethyl cellulose was subjected to addition of a 12% by weight aqueous hydrochloric acid solution in such an amount as to have a concentration of 0.3% by weight on basis of the first hydroxypropylmethyl cellulose. The internal temperature was controlled to 79°C and depolymerization was carried out for 160 minutes to obtain a second (low-polymerization-degree, post-depolymerization) hydroxypropylmethyl cellulose. The second hydroxypropylmethyl cellulose thus obtained was ground using an air stream impact grinder "Jet Mill" (product of Nippon Pneumatic Mfg Co., Ltd.) to obtain intended ground second hydroxypropylmethyl cellulose. The powder properties of the ground second hydroxypropylmethyl cellulose and tablet properties of the tablet obtained by the dry direct tableting in the same manner in Example 1 are shown in Table 1.

### <Example 6>

The intended ground second hydroxypropylmethyl cellulose was obtained in the same manner as in Example 5 except that clearance in a classification zone and grinding pressure were changed in the grinding conditions by using an air stream impact grinder "Jet Mill". The powder properties of the ground second hydroxypropylmethyl cellulose and tablet properties of the tablet obtained by the dry direct tableting in the same manner in Example 1 are shown in Table 1.

### <Comparative Example 1>

The second hydroxypropylmethyl cellulose was obtained in the same manner as in Example 1. The second hydroxypropylmethyl cellulose was used without grinding by using a high-speed rotating impact grinder "Victory Mill". The powder properties of the second hydroxypropylmethyl cellulose and tablet properties of the tablet obtained by the dry direct tableting in the same manner in Example 1 are shown in Table 1.

**Table 1**

| | powder properties | | | | tablet properties | |
|---|---|---|---|---|---|---|
| | specific surface area | average particle size | loose bulk density | viscosity | tablet hardness | disintegration time |
| | (m²/g) | (µm) | (g/mL) | (mPa · s) | (N) | (minutes) |
| Example 1 | 0.51 | 42.0 | 0.41 | 3.18 | 43 | 15 |
| Example 2 | 0.66 | 26.0 | 0.35 | 3.00 | 48 | 20 |
| Example 3 | 2.00 | 19.2 | 0.17 | 4.23 | 70 | 28 |
| Example 4 | 1.24 | 21.3 | 0.25 | 4.45 | 63 | 20 |
| Example 5 | 1.63 | 20.6 | 0.19 | 4.31 | 73 | 8 |
| Example 6 | 2.90 | 10.2 | 0.13 | 4.10 | 94 | 25 |
| Comp.Ex.1 | 0.24 | 78.2 | 0.39 | 3.18 | 34 | 16 |

It is evident from Table 1 that the tablet comprising the hydroxypropylmethyl cellulose obtained in each of Examples 1 to 6 and being obtained by the dry direct tableting showed high hardness even when the amount added was as small as 5% by weight, but significant delay in disintegration time was not observed.

### <Example 7>

The powder mixture below containing the ground second hydroxypropylmethyl cellulose obtained in Example 1 was subjected to dry granulation at roll pressure of 6MPa, roll speed of 4 rpm and screw speed of 5 rpm by using Roller Compactor MINI (product of Freund Corporation).

### Composition of Power Mixture

Acetaminophen fine powder (product of Yamamoto Corporation): 10.0 parts by weight
Lactose hydrate (trade name "Pharmatose 200M", product of DFE Pharma): 79.0 parts by weight
Hydroxypropylmethyl cellulose: 10.0 parts by weight
Light silicic anhydride: 0.5 part by weight
Magnesium stearate: 0.5 part by weight

The obtained granulates were subjected to addition of 0.5 parts by weight of magnesium stearate, mixing for 30 seconds, and tableting under the conditions below to produce tablets. The tablet properties of the produced tablets are shown in Table 2.

### Tableting conditions

Tableting machine: rotary tableting machine (trade name "VIRGO"; product of Kikusui Seisakusho)
Tablet size: 200 mg/tablet, a diameter (D) of 8 mm and radius of curvature (R) of 12 mm

Tableting pressure: 10 kN
Tableting speed: 20 rpm

### <Example 8>

The dry granulation was carried out in the same manner as in Example 7 except that the hydroxypropylmethyl cellulose obtained in Example 2 was used. The obtained granulates were subjected to addition of 0.5 parts by weight of magnesium stearate, mixing for 30 seconds, and tableting under the conditions below to produce tablets. The tablet properties of the produced tablets are shown in Table 2.

### <Comparative Example 2>

The dry granulation was carried out in the same manner as in Example 7 except that the hydroxypropylmethyl cellulose obtained in Comparative Example 1 was used. The obtained granulates were subjected to addition of 0.5 parts by weight of magnesium stearate, mixing for 30 seconds, and tableting under the conditions below to produce tablets. The tablet properties of the produced tablets are shown in Table 2.

**Table 2**

| | tablet properties | |
|---|---|---|
| | tablet hardness | disintegration time |
| | (N) | (minutes) |
| Example 7 | 41 | 22 |
| Example 8 | 42 | 23 |
| Comp.Ex.2 | 35 | 21 |

It is evident from Table 2 that the tablet comprising the hydroxypropylmethyl cellulose obtained in each of Examples 7-8 and being obtained by the dry granulation tableting showed high hardness in comparison with the result of Comparative Example 2 , but delay in disintegration time was not observed.

## Claims

1. A hydroxyalkylalkyl cellulose for tableting having a specific surface area of from 0.5 to 5.0 m²/g as measured by the BET method.

2. The hydroxyalkylalkyl cellulose for tableting according to claim 1,
wherein a 2% by weight aqueous solution thereof has a viscosity at 20°C of from 1 to 15 mPa·s.

3. The hydroxyalkylalkyl cellulose for tableting according to claim 1 or 2, having an average particle size of from 5 to 70 µm.

4. The hydroxyalkylalkyl cellulose for tableting according to any one of claims 1 to 3, having a loose bulk density of from 0.05 to 0.5 g/mL.

5. The hydroxyalkylalkyl cellulose for tableting according to any one of claims 1 to 4, wherein the hydroxyalkylalkyl cellulose is hydroxypropylmethyl cellulose.

6. A solid preparation comprising the hydroxyalkylalkyl cellulose for tableting as claimed in any one of claims 1 to 5.

7. A method for producing a hydroxyalkylalkyl cellulose for tableting having a specific surface area of from 0.5 to 5.0 m²/g as measured by the BET method, comprising the steps of:
bringing pulp into contact with an alkali metal hydroxide solution to obtain an alkali cellulose,
reacting the alkali cellulose with an etherifying agent to obtain a first hydroxyalkylalkyl cellulose,
grinding the first hydroxyalkylalkyl cellulose, and
subjecting the ground first hydroxyalkylalkyl cellulose to hydrolysis in the presence of an acid catalyst or oxidative degradation in the presence of an oxidant for depolymerization to obtain a second hydroxyalkylalkyl cellulose.

8. A method for producing a solid preparation comprising:
the steps comprised by the method as claimed in claim 7 to produce a hydroxyalkylalkyl cellulose for tableting; and
a step of tableting the hydroxyalkylalkyl cellulose by dry direct tableting or dry granulation tableting.
